Europäisches Patentamt

European Patent Office

Office européen des brevets

(11) Veröffentlichungsnummer: **0 200 156 B1**

(19)

### (12) EUROPÄISCHE PATENTSCHRIFT

(45) Veröffentlichungstag der Patentschrift: **22.04.92**

(21) Anmeldenummer: **86105647.1**

(22) Anmeldetag: **24.04.86**

(51) Int. Cl.⁵: **A61K 33/40**

(54) **Verwendung einer wässrigen Chloritmatrix-Lösung zur Herstellung von Arzneimitteln zur intravenösen Behandlung von Tumoren.**

(30) Priorität: **02.05.85 DE 3515748**

(43) Veröffentlichungstag der Anmeldung:
**05.11.86 Patentblatt 86/45**

(45) Bekanntmachung des Hinweises auf die
Patenterteilung:
**22.04.92 Patentblatt 92/17**

(84) Benannte Vertragsstaaten:
**AT BE CH DE FR GB IT LI LU NL SE**

(56) Entgegenhaltungen:
**EP-A- 0 093 875**

**CHEMICAL ABSTRACTS, Band 86, 1977, Seite 379, Zusammenfassung Nr. 161332a, Columbus, Ohio, US; & SU-A-68 443 (I.F. SPORYK-HIN) 25-02-1977**

(73) Patentinhaber: **OXO Chemie GmbH
Im Neuenheimer Feld 517
W-6900 Heidelberg(DE)**

(72) Erfinder: **Kühne, Friedrich W., Dr.
Bergstrasse 72
W-6900 Heidelberg(DE)**
Erfinder: **Stahl, Kurt-Wilhelm, Prof. Dr. Dr.
Goethestrasse 69
W-7800 Freiburg(DE)**

(74) Vertreter: **Grussdorf, Jürgen, Dr. et al
Patentanwälte Zellentin & Partner Rubensstrasse 30
W-6700 Ludwigshafen(DE)**

**Beschreibung**

Die Erfindung betrifft die Verwendung einer Chloritmatrix-Lösung zur Herstellung von Arzneimitteln zur intravenösen Behandlung von Tumoren.

Bei der herkömmlichen chemotherapeutischen Behandlung von Tumoren muss zur Erreichung einer objektivierbaren Tumorregression ein erhebliches, die Lebensqualität beeinträchtigendes Nebenwirkungsrisiko in Kauf genommen werden.

Aufgabe der Erfindung ist es, mit Hilfe einer Zubereitung dieses Nebenwirkungsrisiko chemotherapeutischer und radiotherapeutischer Behandlungen von Tumoren zu verringern, bei gleichzeitiger Verbesserung oder mindestens Beibehaltung der therapeutischen Wirksamkeit der Behandlungen.

Gelöst wird diese Aufgabe durch die Verwendung einer Zubereitung, bestehend aus einer wässrigen Lösung einer chemisch stabilisierten Chloritmatrix zur intravenösen Applikation bei Tumorbehandlungen.

Die Verwendung der Zubereitung kann kombiniert werden mit Radiotherapie, mit Chemotherapie, mit Radio- und Chemotherapie. sowie mit Chemotherapie und Hyperthermie.

Eine Chloritmatrix mit aktiviertem Sauerstoff ist aus der DE-OS 32 13 389 bekannt und zur Behandlung von Melanomen durch parenterale Applikation und als Wundbehandlungsmittel bereits erfolgreich eingesetzt. Es wurde nun überraschenderweise gefunden, daß eine chemisch stabilisierte Chloritmatrix mit guten Ergebnissen auch intravenös zu Tumorbehandlungen verwendet werden. kann, wobei die Matrix durch Sauerstoff oder ein anderes Element der Gruppen VI a + b des Periodischen Systems oder in einer anderen Weise stabilisiert sein kann. Es ist bekannt, daß die Sauerstoffversorgung von malignen Tumoren die Wirksamkeit verschiedener therapeutischer Maßnahmen, wie ionisierender Strahlen oder bestimmter Chemotherapeutika sehr stark beeinflussen kann. Versuche haben gezeigt, daß bei intravenöser Verabreichung einer isotonen Lösung der Chloritmatrix ein Pasteur-Effekt erreicht werden kann, wobei die Wirkung der Chloritmatrix direkt und/oder indirekt ist. Eine direkte und indirekte Wirkung ist gegeben, wenn der Stabilisator der Chloritmatrix Sauerstoff ist. Indirekt ist die Wirkung, wenn mit einem anderen Element der Gruppe VI a + b des Periodischen Systems oder auf andere Weise, z. B. einem hohen pH-Wert, stabilisiert ist.

Es wurde gefunden, daß die Chloritmatrix praktisch als Biochemikalie bereits im mikromolaren Bereich und darunter wirkt und deshalb auch nicht in Überkonzentrationen angeboten werden muß, um seine Wirkung zu entfalten. Dabei wird angenommen, daß eine aktivierte nicht toxische Sauerstoffspezies gebildet wird, durch die eine Erhöhung der Durchblutung in peripheren, hypoxischen Bereichen durch Vasodilatation sowie eine Sauerstoff-Sparmaßnahme in den Mitochondrien erreicht wird. Diese Wirkung dürfte zu einer Erhöhung der Sauerstoffwerte im Gewebe bzw. in den Körperflüssigkeiten nach der Zugabe der Chloritmatrix beitragen.

Wird die Chemotherapie und die Radiotherapie mit einer Chloritmatrix-Behandlung kombiniert, können zur Erreichung gleicher Ergebnisse die Dosierungen chemotherapeutischer Mittel sowie der Bestrahlungen - und damit das Nebenwirkungsrisiko - wesentlich verringert werden.

Experimentelle Ergebnisse zur antitumorösen Wirksamkeit von stabilisierter Chloritmatrix.

Zur Verwendung kam eine isotone Lösung von sauerstoffstabilisierter Chloritmatrix, welche $2,8 \times 10^2$ Oxidationseinheiten gemessen über die gaschromatographische Bestimmung freigesetzten Ethylens aus einem geeigneten Indikatormolekül enthält. Versuche an mehreren Transplantationstumormodellen der Maus bzw. der Ratte haben gezeigt, daß die Chloritmatrix-Lösung in Abhängigkeit von der Dosierung (Dosierungen: 0,17, 0,34 und 0,68 ml Chloritmatrix-Lösung/kg Körpergewicht, gegeben 1 x täglich parenteral) das Tumorwachstum verlangsamt. Die hemmende Wirkung bezieht sich bei metastasierenden soliden Tumoren nicht nur auf den Primärtumor, sondern auch auf die Sekundärabsiedelungen (Metastasen).

Die Testung auf antitumoröse Wirksamkeit brachte bei folgenden Transplantationstumoren positive Ergebnisse: Lewis Lung, Adenokarzinom der C 57 Bl/6 Maus, Harding-Passey-Sarkom der C 57 Bl/6 Maus, Friend-Virusleukämie der NMRI-Maus sowie L 5222 Leukämie der BD IX-Ratte. Bei allen hier genannten Tumoren fiel auf, daß unter der Behandlung mit der stabilisierten Chloritmatrix die Gewichtsreduktion zwar undramatischer verlief als bei dem mit Cyclophosphamid behandelter Positivkontrollen, daß aber die mit Chloritmatrix-Lösung behandelten Tiere ein deutlich besseres und aktiveres Allgemeinbefinden zeigten als die mit dem cytotoxischen Chemotherapeutikum therapierten Tiere.

Stabilisierte Chloritmatrices werden durch Komplexierung mit macrocyclische Tetrapyrrole enthaltenden Biomolekülen zu elektronenaffinen Verbindungen aktiviert. Von elektronenaffinen Verbindungen ist bekannt, daß sie strahlensensibilisierend bei der radiotherapeutischen Behandlung von bösartigen Tumoren wirken (Literaturzitat W. Porschen, K. Gewehr, L.E. Feinendegen, Med. Physik Band 2 Seite 467-479/1976). Diese pharmakologische Eigenschaft konte durch erste positive Befunde in der Strahlentherapie nach parenteraler Vorbehandlung mit der Chloritmatrix-Lösung bei Patienten mit strahlentherapeutisch schlecht ansprechbaren Tumoren bestätigt werden.

Die tierexperimentell durchgeführten therapeutischen Versuche an Transplantationstumoren haben gezeigt, daß die antitumoröse Wirksamkeit von stabilisierten Chloritmatrices kein cytotoxischer bzw. cytostatischer Effekt ist. Vielmehr beeinflussen die stabilisierten Chloritmatrices einerseits das körpereigene Abwehrsystem, andererseits zeigen die mit der L 5222 und der Friendvirus-Leukämie durchgeführten Versuche, daß die Chloritmatrices eine revertierende Wirkung auf den bösartigen Zellphänotyp der Tumoren hat. Diese im Vergleich zu den herkömmlichen Chemotherapeutika völlig anders gearteten Angriffspunkte führen zu dem Schluß, daß eine Zusatzbehandlung mit stabilisierten Chloritmatrices den antitumorösen Effekt der klassischen Chemotherapeutika potenzieren kann.

## Patentansprüche

1. Verwendung einer wässrigen Lösung einer chemisch stabilisierten Chloritmatrix zur Herstellung von Arzneimitteln zur intravenösen Applikation bei Tumorbehandlungen.

2. Verwendung nach Anspruch 1, wobei die Tumorbehandlung auch Radiotherapie einschließt.

3. Verwendung nach Anspruch 1, wobei die Tumorbehandlung auch Chemotherapie einschließt.

4. Verwendung nach Anspruch 1, wobei die Tumorbehandlung auch Radiotherapie und Chemotherapie einschließt.

5. Verwendung nach Anspruch 1, wobei die Tumorbehandlung auch Chemotherapie und Hyperthermie einschließt.

## Claims

1. Use of an aqueous solution of a chemically stabilised chlorite matrix for the preparation of medicaments for intravenous administration in the case of tumour treatments.

2. Use according to claim 1, whereby the tumour treatment also includes radiotherapy.

3. Use according to claim 1, whereby the tumour treatment also includes chemotherapy.

4. Use according to claim 1, whereby the tumour treatment also includes radiotherapy and chemotherapy.

5. Use according to claim 1, whereby the tumour treatment also includes chemotherapy and hyperthermia.

## Revendications

1. Utilisation d'une solution aqueuse d'une matrice dc chlorite chimiquement stabilisée pour la préparation de médicaments à appliquer par voie intraveineuse dans des traitements de tumeurs.

2. Utilisation suivant la revendication 1, dans laquelle le traitement de tumeurs comprend également une radiothérapie.

3. Utilisation suivant la revendication 1, dans laquelle le traitement de tumeurs comprend également une chimiothérapie.

4. Utilisation suivant la revendication 1, dans laquelle le traitement de tumeurs comprend également une radiothérapie et une chimiothérapie.

5. Utilisation suivant la revendication 1, dans laquelle le traitement de tumeurs comprend également une chimiothérapie et une hyperthermie.